# FASCICULE DE BREVET EUROPEEN

(11) **EP 0 581 649 B1**
(45) Date de publication et mention de la délivrance du brevet: **28.10.1998**
(21) Numéro de dépôt: 93401894.6
(22) Date de dépôt: 22.07.1993
(51) Int. Cl.: C07J 9/00, C07J 31/00, C12P 41/00

(54) **Nouveau procédé de préparation de composés stéroides 20-céto 21(S)-hydroxy et des intermédiaires**
Neues Verfahren zur Herstellung von 20-keto 21(S)-hydroxy-Steroiden und Zwischenprodukten davon
New method of production of 20-keto 21(S)-hydroxy steroids and intermediates thereof

(30) Priorité: 23.07.1992 FR 9209077
(43) Date de publication de la demande: 02.02.1994
(73) Titulaire: ROUSSEL UCLAF, 93230 Romainville (FR)
(72) Inventeur: Buendia, Jean, F-94170 Le Perreux sur Marne (FR); Godard, Jean-Yves, F-93340 Le Raincy (FR); Mackiewicz, Philippe, F-93190 Livry Gargan (FR); Richard, Christian, F-93110 Rosny sous Bois (FR)
(74) Mandataire: Vieillefosse, Jean-Claude

(56) Documents cités:
- EP-A- 0 007 823
- NUCLEAR MEDICINE AND BIOLOGY vol. 17, no. 3 , 1990 , OXFORD GB pages 309 - 319 M. G. POMPER ET AL 'Target Tissue Uptake Selectivity of Three Fluorine-Substituted Progestins: Potential Imaging Agents for Receptor Positive Breast Tumors'
- CHEMICAL ABSTRACTS, vol. 108, no. 17, 25 Avril 1988, Columbus, Ohio, US; abstract no. 148914, H. OTA ET AL 'Manufacture of Optically Active alpha-Hydroxyketones and Their Carboxylic Acid Esters by Esterase Containing Microorganisms' page 604 ;colonne 2 ;

## Description

La présente invention concerne un nouveau procédé de préparation des composés stéroïdes 20-céto 21(S)-hydroxy et des intermédiaires.

L'invention a ainsi pour objet un procédé de préparation des composés de formule (I) : 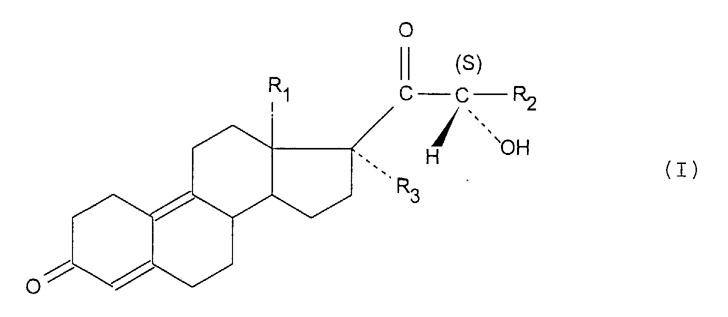 dans laquelle :
. R₁ représente un radical alkyle renfermant de 1 à 3 atomes de carbone,
. R₂ un radical alkyle renfermant de 1 à 12 atomes de carbone,
. R₃ un radical alkyle renfermant de 1 à 4 atomes de carbone
caractérisé en ce que l'on traite un composé de formule (II) : 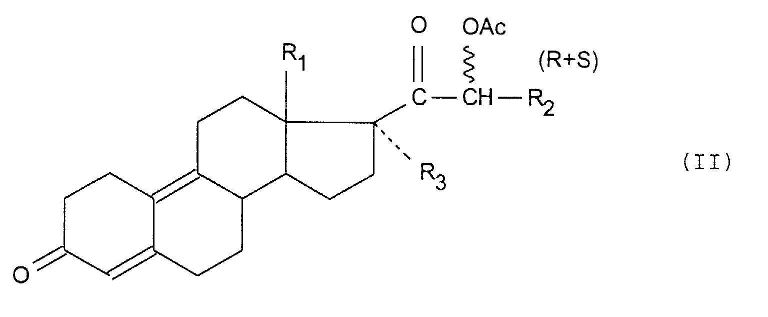 dans laquelle R₁, R₂ et R₃ sont définis comme précédemment, sous forme d'un mélange d'isomères en position 21, par une estérase ou une lipase, pour obtenir un mélange du composé de formule (III) : 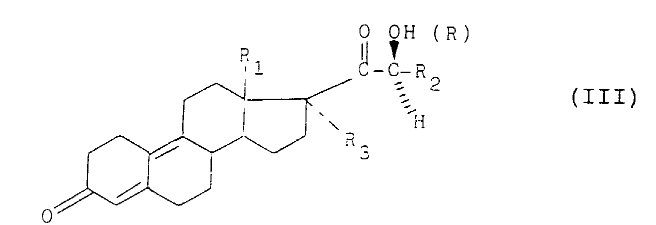 et du composé de formule (II₁) : 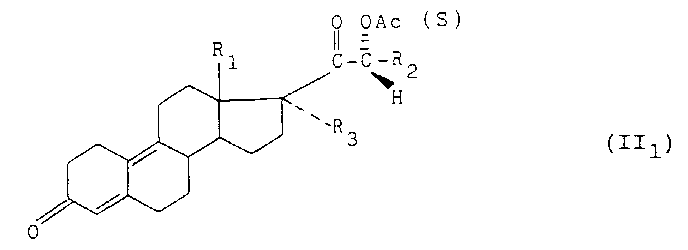 que l'on traite par un réactif de formule (A) :

Hal-SO₂-R (A)

dans laquelle Hal représente un atome d'halogène et R représente un radical alkyle renfermant de 1 à 6 atomes de carbone, un radical phényle éventuellement substitué par un ou plusieurs radicaux alkyle renfermant de 1 à 3 atomes de carbone, ou un radical -CX_{3,} dans lequel X représente un atome de fluor, de chlore ou de brome, pour obtenir un mélange du composé de formule : 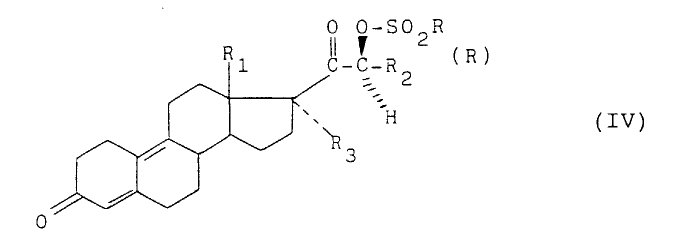 et du composé de formule (II₁) ci-dessus, que soit l'on soumet à l'action d'un acétate alcalin, pour obtenir le composé de formule (II₁) définie ci-dessus, que l'on soumet à une solvolyse en milieu basique, soit l'on soumet à l'action d'un agent hydroxylant basique et nucléophile, pour obtenir le composé de formule (I) attendu.

R₁ peut représenter un radical méthyle, éthyle ou propyle, méthyle étant préféré.

R₂ peut notamment représenter un radical méthyle, éthyle, propyle, isopropyle, n-butyle, isobutyle, n-pentyle, n-hexyle, 2-méthylpentyle, 2,3-diméthylbutyle, n-octyle, ou 2,2-diméthyl-hexyle.

R₃ peut notamment représenter un radical méthyle, éthyle ou n-propyle.

L'agent d'hydrolyse diastéréosélectif du mélange d'esters de formule (II) est une estérase ou une lipase, par exemple l'une de celles connues sous les noms de lipase porcin pancreatic, PS "Amano", A₁ "Enzymatix", liposyme 10000 L, N "Amano", F₈ "Enzymatix", F₃ "Enzymatix", B₁ "Enzymatix" ou lipase Pseudomonas fluorescens, la lipase connue sous l'appellation PS "Amano" étant préférée. Les dénominations "Amano" et "Enzymatix" sont des marques commerciales déposées. On opère à pH neutre ou, de préférence, faiblement acide, de l'ordre de 5, obtenu par l'utilisation d'un tampon aqueux approprié et, le cas échéant, l'addition d'une base. On opère à température ambiante ou, de préférence, légèrement supérieure, de l'ordre de 40°C. On peut opérer en présence d'un cosolvant, lequel peut être un alcane tel que l'hexane ou, de préférence, un alcanol inférieur tel que le n-butanol, le propanol ou l'isopropanol.

Le réactif de formule (A) est de préférence un chlorure ou un bromure de méthanesulfonyle, d'éthanesulfonyle, de phénylsulfonyle, de p-toluènesulfonyle ou de trifluorométhylsulfonyle et l'on opère en présence d'un agent basique qui est de préférence une amine telle que la triéthylamine, la pyridine, la diméthylaminopyridine, ou une base minérale telle qu'un hydroxyde ou un carbonate alcalin, au sein d'un solvant organique, par exemple un solvant halogéné tel que le chlorure de méthylène, le chloroforme ou le dichloréthane, ou un hydrocarbure aromatique ou saturé, tel que le benzène, le toluène ou le cyclohexane.

L'inversion du sulfonate de formule (IV) au sein d'un mélange avec le composé de formule (II₁) peut être réalisée par l'intermédiaire d'une substitution nucléophile de type SN₂, en utilisant un acétate alcalin, plus particulièrement l'acétate de sodium ou l'acétate de potassium. On opère par chauffage au sein d'un solvant organique polaire aprotique tel que le bis(2-méthoxy éthyl) éther, le diméthylformamide diméthylacétamide, le diméthylsulfoxyde ou la diméthylpyrrolidone.

La solvolyse finale est de préférence une alcoolyse effectuée en présence de soude ou de potasse. L'alcool utilisé est un alcanol inférieur et de préférence le méthanol ou l'éthanol.

La solvolyse peut encore être une hydrolyse mettant en oeuvre par exemple les bases mentionnées ci-dessus.

L'inversion du sulfonate peut aussi être réalisée par l'action d'un agent hydroxylant basique et nucléophile qui peut être par exemple un hydroxyde alcalin ou alcalino-terreux, tel que l'hydroxyde de sodium, l'hydroxyde de potassium, l'hydroxyde de calcium ou l'hydroxyde de baryum. On opère dans un solvant tel que les mélanges de tétrahydrofurane, bis(2-méthoxyéthyl) éther, triéthylèneglycol diméthyl éther, diméthylformamide, diméthylsulfoxyde, diméthylacétamide avec l'eau.

L'invention a tout particulièrement pour objet un procédé tel que décrit précédemment, caractérisé en ce que l'on utilise au départ un composé de formule (II) dans laquelle R₁, R₂ et R₃ représentent un radical méthyle.

L'invention a enfin pour objet, à titre de composés industriels nouveaux, les mélanges des composés de formules (II₁) et (III) et des composés de formules (II₁) et (IV), telles que définies précédemment.

Les composés de formule (I) sont doués d'activité progestomimétique et anti-estrogène et sont décrits dans le brevet européen n° 7823.

Les composés de départ de formule (II) sont décrits dans ce même brevet.

Les différentes enzymes mentionnées plus haut sont des produits du commerce, disponibles par exemple auprès des Sociétés Amano Pharmaceutical CO Ltd, Enzymatix Ltd, Sigma Chimie SARL ou encore Novo Nordisk Bio Industrie.

Les exemples suivants illustrent l'invention sans toutefois la limiter.

### Exemple 1: 17α-méthyl 17β-(2S-hydroxy 1-oxo propyl) estra 4,9-dièn-3-one.

Stade A : Mélange de 17α-méthyl 17β-(2R-hydroxy 1-oxo propyl) estra-4,9-dièn-3-one et de 17α-méthyl 17β-(2S-acétoxy 1-oxo propyl) estra-4,9-dièn-3-one.
On mélange 5 g de 17a-méthyl 17β-(2R,S-acétoxy 1-oxo propyl) estra-4,9-dièn-3-one, 5 g de lipase PS "Amano", 7 cm³ de n-butanol et 18 cm³ de Tampon pH=5. On maintient la température à 37°C et on maintient le pH à 5 par addition de soude 1N. Après 144 heures, on rajoute 3,75 cm³ de Tampon pH 5, 1,25 cm³ de n-butanol et 1, 25 g de lipase PS "Amano". Après 168 heures, on extrait au chlorure de méthylène, lave la phase organique à l'eau, sèche et évapore le solvant. On obtient 4,63 g de mélange attendu renfermant environ 50 % de chacun des constituants.
Spectre RMN CDCl₃ (300 MHz)
- Mélange 50/50 de 2 constituants
   1) 0,81 (s) : 18-CH₃ ; 1,16 (s) : 17-CH₃ ; 1,33 (d) CH₃-CH 3,71 (d) : OH- ; 4,56 (quintuplet) : -CH-OH ; 5,68 (s) : H₄.
   2) 0,82 (s) : 18-CH₃ ; 1,21 (s) : 17-CH₃ ; 1,38 (d) : CH₃-CH- ; 2,11 (s) : O-Ac ; 5,42 (q) : CH₃-CH- ; 5,68 (s) : H₄.

Les proportions respectives des deux constituants du mélange ont été également déterminées par HPLC comparativement aux constituants purs décrits et caractérisés dans le brevet européen n° 7823 (Exemple 2).
Stade A' : Mélange de 17α-méthyl 17β-(2R-hydroxy 1-oxo propyl) estra-4,9-dièn-3-one et de 17α-méthyl 17β-(2S-acétoxy 1-oxo propyl) estra-4, 9-dièn-3-one.
On mélange 0,1 g de 17α-méthyl 17β-(2R,S-acétoxy 1-oxo propyl) estra-4,9-dièn-3-one, 2 cm³ de Tampon phosphate pH 7, 20 mg d'enzymefigurant dans le tableau ci-dessous. On agite le mélange (300 tours/min) et contrôle le pH afin de le maintenir entre 5 et 8.
On a obtenu après agitation pendant 64 à 137 heures le mélange attendu, l'ester (R) étant hydrolysé dans les proportions figurant dans le tableau.
La réaction est suivie par chromatographie sur couche mince en éluant au mélange toluène-acétate d'éthyle (1-1). La composition du mélange final est déterminée par HPLC sur colonne PARTISIL n° 2102 D, en éluant au mélange hexane-chlorure de méthylène-dioxanne (8-1-1). On obtient les résultats suivants :

| Enzyme Utilisée | % d'alcools R et S dans le milieu |
|---|---|
| Lipase PS Amano | > 99,5 % de (R) |
| Lipase A₁ enzymatix | 85 % (R) - 15 % (S) |
| Liposyme 10 000 L | 90 % (R) - 10 % (S) |
| Lipase B₁ enzymatix | > 95 % de (R) |
| Pseudomonas fluorescens | > 90 % de (R) |

Stade B : Mélange de 17α-méthyl 17β-(2R-méthyl sulfonyloxy 1-oxopropyl) estra-4,9-dièn-3-one et de 17α-méthyl 17β-(2S-acétoxy 1-oxopropyl) estra-4,9-dièn-3-one.
On mélange sous gaz inerte 2 g de produit obtenu au stade A et 10 cm³ de chlorure de méthylène, puis ajoute à la solution obtenue 0,8 cm³ de triéthylamine. On refroidit à 0°/-2°C, puis ajoute lentement en maintenant la température à 0°/-2°C, 0,35 cm³ de chlorure de méthane sulfonyle. On agite pendant 1 heure, puis verse le mélange dans 5 cm³ d'eau. On extrait au chlorure de méthylène, lave la phase organique à l'eau, sèche et évapore le solvant. On obtient 2,35 g de mélange attendu, composé approximativement de 46 % d'acétate (S) et de 54 % de mésylate (R). (Détermination par HPLC Colonne PARTISIL/Hexane-chlorure de méthylène-dioxane 80/10/10).
Spectre RMN CDCl₃ (300 MHz)
- Mélange 50/50 de 2 constituants
   1) 0,90 (s) : 18-CH₃ ; 1,56 (d) : CH₃-CH- ; 3,16 (s) O-SO₂-CH₃.
   2) 0,82 (s) : 18-CH₃ ; 1,39 (d) : CH₃-CH- ; 2,11 (s) : OAc ; et, en outre, 1,18 (s) et 1,20 (s) : 17-CH₃ ; 5,42 (q) et 5,52 (q) : -CO-CH-CH₃ 5,68 (s) : H₄.

Stade C : 17α-méthyl 17β-(2S-acétoxy 1-oxo propyl) estra-4,9-dièn-3-one.
On mélange sous gaz inerte 1 g de produit obtenu au stade B et 7 cm³ de bis(2-méthoxy éthyl) éther. On ajoute à la solution 0,38 g d'acétate de potassium puis chauffe à 90°C pendant 22 heures. On refroidit à température ambiante, verse dans 20 cm³ d'eau et agite pendant 4 heures. On essore le produit et le rince à l'eau puis le sèche. On obtient 0,77 g de produit attendu renfermant environ 94 % d'isomère (S). (Détermination par HPLC comme au Stade B).
Spectre RMN CDCl₃ (300 MHz)
- Présence à 95 % environ du constituant caractérisé comme suit :
   0,82 (s) : 18-CH₃ ; 1,20 (s) : 17-CH₃ ; 1,39 (d) et
   5,42 (q) : CH₃-CH- ; 2,11 (s) : O-Ac ; 5,69 (s) : H₄.

Stade D : : 17α-méthyl 17β-(2S-hydroxy 1-oxo propyl) estra-4,9-dièn-3-one.
On mélange 0,5 g de produit obtenu au stade C, 4 cm³ de méthanol et 0,5 cm³ de potasse méthanolique à 0,06 g/5 cm³. On agite la solution pendant 4 heures à 20°C puis neutralise par addition de 0,7 cm³ d'acide chlorhydrique 0,1 N. On ajoute 5 cm³ de chlorure de méthylène et 3 cm³ d'eau, décante et réextrait la phase aqueuse au chlorure de méthylène. On sèche les phases organiques réunies et évapore le solvant. On obtient 0,427 g de produit attendu renfermant 94 % d'isomère (S). (Détermination par HPLC comme aux stades B et C).
Spectre RMN CDCl₃ (300 MHz)
- Présence à 95 % environ du composé 21(S) tel que caractérisé à l'exemple 1 du brevet européen n° 7823 (isomère A).

### Exemple 2 : 17α-méthyl 17β-(2S-hydroxy 1-oxo propyl) estra4,9-dièn-3-one.

On mélange sous gaz inerte 100 mg de produit obtenu au stade B de l'exemple 1,3 cm³ de solvant et 100 mg de base. On chauffe le mélange sous agitation en suivant la réaction en HPLC. (Colonne Partisil - éluant : hexane-chlorure de méthylène-dioxanne 8.1.1).

Les conditions précises et résultats figurent sur le tableau ci-après.

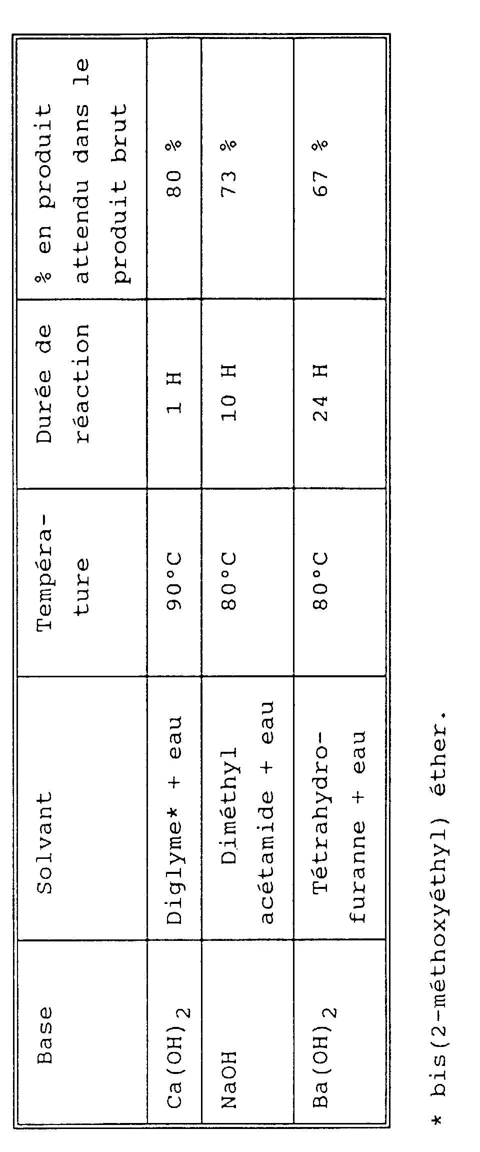

## Revendications

1. Procédé de préparation des composés de formule (I) : 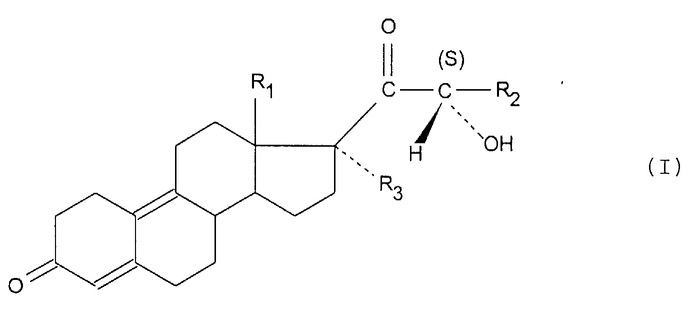 dans laquelle :
. R₁ représente un radical alkyle renfermant de 1 à 3 atomes de carbone,
. R₂ un radical alkyle renfermant de 1 à 12 atomes de carbone,
. R₃ un radical alkyle renfermant de 1 à 4 atomes de carbone
caractérisé en ce que l'on traite un composé de formule (II) : 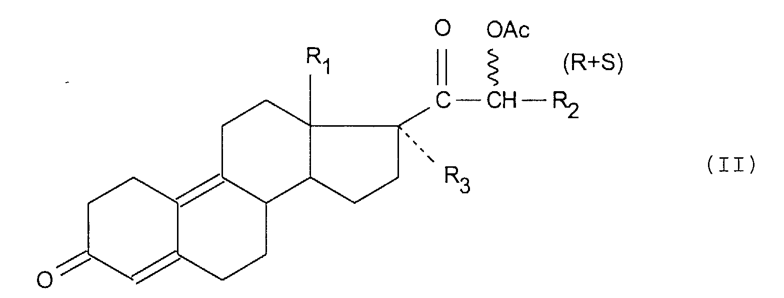 dans laquelle R₁, R₂ et R₃ sont définis comme précédemment, sous forme d'un mélange d'isomères en position 21, par une estérase ou une lipase, pour obtenir un mélange du composé de formule (III) : 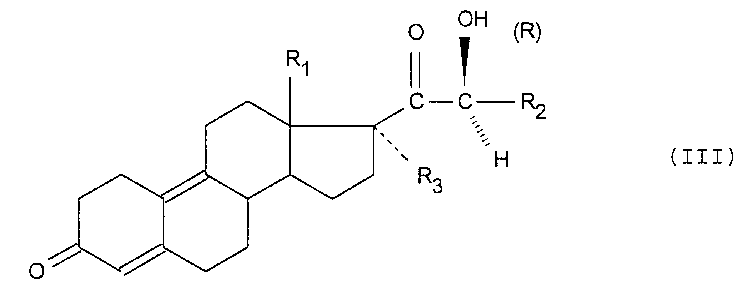 et du composé de formule (II₁) : 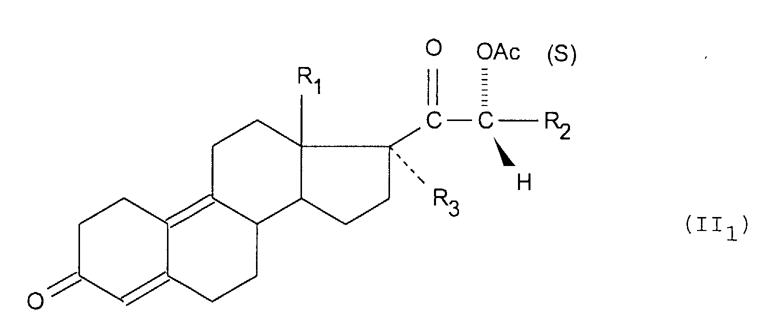 que l'on traite par un réactif de formule (A) :
Hal-SO₂-R (A)
dans laquelle Hal représente un atome d'halogène et R représente un radical alkyle renfermant de 1 à 6 atomes de carbone, un radical phényle éventuellement substitué par un ou plusieurs radicaux alkyle renfermant de 1 à 3 atomes de carbone, ou un radical -CX₃, dans lequel X représente un atome de fluor, de chlore ou de brome, pour obtenir un mélange du composé de formule : 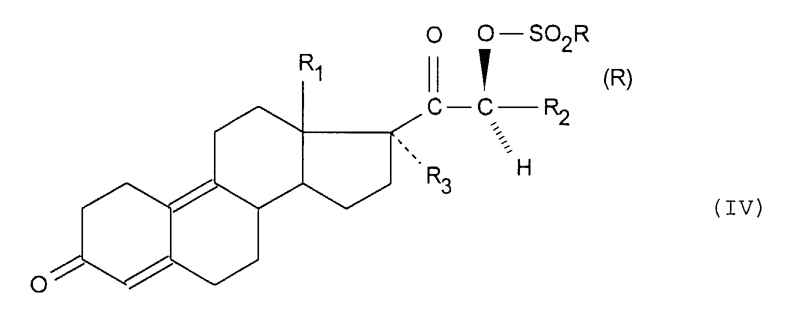 et du composé de formule (II₁) ci-dessus, que soit l'on soumet à l'action d'un acétate alcalin, pour obtenir le composé de formule (II₁) définie ci-dessus, que l'on soumet à une solvolyse en milieu basique, soit l'on soumet à l'action d'un agent hydroxylant basique et nucléophile, pour obtenir le composé de formule (I) attendu.

2. Procédé selon la revendication 1, caractérisé en ce que l'enzyme est choisie dans le groupe constitué par la lipase Porcin Pancreatic, PS "Amano", A₁ "Enzymatix", liposyme 10000 L, N "Amano", F₈ "Enzymatix", F₃ "Enzymatix", B₁ "Enzymatix" et la lipase Pseudomonas fluorescens.

3. Procédé selon l'une quelconque des revendications 1 et 2, caractérisé en ce que l'agent d'hydrolyse diastéréosélectif est la lipase PS "Amano".

4. Procédé selon l'une quelconque des revendications 1 à 3, caractérisé en ce que l'on opère à pH neutre ou faiblement acide.

5. Procédé selon l'une quelconque des revendications 1 à 4, caractérisé en ce que l'on utilise un réactif de formule (A) choisi dans le groupe constitué par les chlorures et bromures de méthane sulfonyle, d'éthane sulfonyle, de phényl sulfonyle, de p-toluène sulfonyle et de trifluorométhyl sulfonyle.

6. Procédé selon l'une quelconque des revendications 1 à 5, caractérisé en ce que l'acétate alcalin utilisé est l'acétate de sodium ou de potassium.

7. Procédé selon l'une quelconque des revendications 1 à 6, caractérisé en ce que la solvolyse finale est effectuée par la soude ou la potasse au sein d'un alcanol inférieur.

8. Procédé selon l'une quelconque des revendications 1 à 5, caractérisé en ce que l'agent hydroxylant basique et nucléophile est choisi dans le groupe constitué par l'hydroxyde de sodium, l'hydroxyde de potassium, l'hydroxyde de calcium et l'hydroxyde de baryum.

9. Procédé selon l'une quelconque des revendications 1 à 8, caractérisé en ce que l'on utilise au départ un composé de formule (II) dans laquelle R₁, R₂ et R₃ représentent un radical méthyle.

10. A titre de composés industriels nouveaux, les mélanges des composés de formules (II₁) et (III) et des composés de formules (II₁) et (IV), telles que définies à la revendication 1.

## Patentansprüche

1. Verfahren zur Herstellung von Verbindungen der Formel (I) 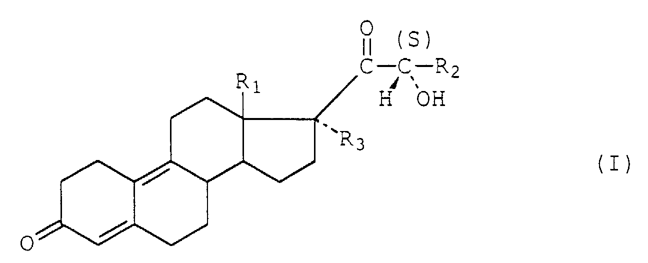 in der
. R₁ einen Rest Alkyl mit 1 bis 3 Kohlenstoffatomen,
. R₂ einen Rest Alkyl mit 1 bis 12 Kohlenstoffatomen,
. R₃ einen Rest Alkyl mit 1 bis 4 Kohlenstoffatomen darstellen,
dadurch gekennzeichnet, daß man eine Verbindung der Formel (II) 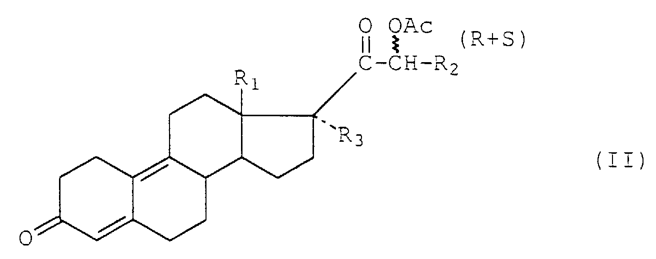 in der R₁, R₂ und R₃ wie vorstehend definiert sind, in Form einer Mischung der Isomeren in Position 21 mit einer Esterase oder einer Lipase behandelt, um eine Mischung der Verbindung der Formel (III) 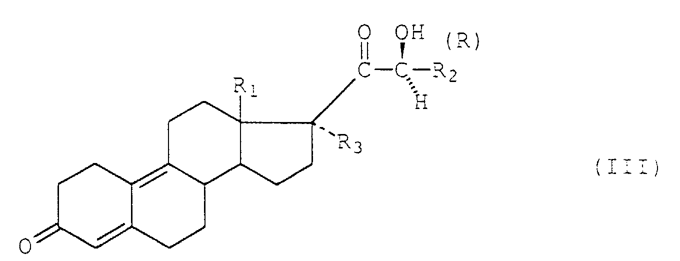 und der Verbindung der Formel (II₁) 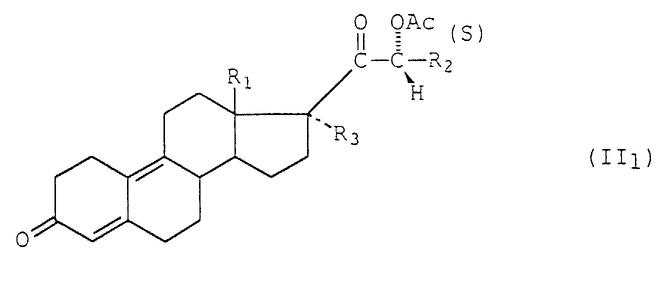 zu erhalten, die man mit einem Reaktanden der Formel (A)
Hal-SO₂-R (A)
behandelt, in der Hal ein Halogenatom darstellt und R einen Rest Alkyl mit 1 bis 6 Kohlenstoffatomen, einen Rest Phenyl, gegebenenfalls substituiert durch einen oder mehrere Reste Alkyl mit 1 bis 3 Kohlenstoffatomen, oder einen Rest -CX₃ bedeutet, worin X ein Atom von Fluor, Chlor oder Brom ist, um eine Mischung der Verbindung der Formel (IV) 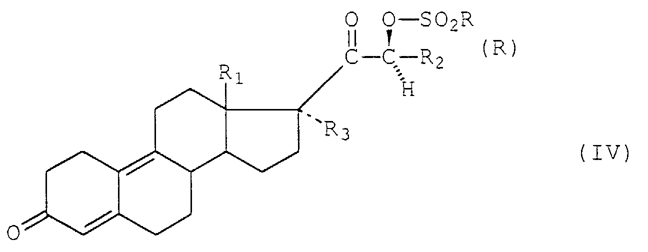 und der obigen Verbindung der Formel (II₁) zu erhalten, die man der Einwirkung eines Alkaliacetates unterzieht, um die wie oben definierte Verbindung der Formel (II₁) zu erhalten, die man dann entweder einer Solvolyse im basischen Medium oder der Einwirkung eines basischen und nucleophilen hydroxylierenden Mittels unterwirft, um die erwartete Verbindung der Formel (I) zu erhalten.

2. Verfahren nach Anspruch 1, dadurch gekennzeichnet, daß das Enzym aus der durch die Lipase Porcin Pancreatic, PS. "Amano", A₁ "Enzymatix", Liposym 10000 L, N "Amano", F₈ "Enzymatix", F₃ "Enzymatix", B₁ "Enzymatix" und die Lipase Pseudomonas fluorescens gebildeten Gruppe gewählt wird.

3. Verfahren nach irgendeinem der Ansprüche 1 und 2, dadurch gekennzeichnet, daß das Mittel zur diastereoselektiven Hydrolyse die Lipase PS "Amano" ist.

4. Verfahren nach irgendeinem der Ansprüche 1 bis 3, dadurch gekennzeichnet, daß man bei einem neutralen oder schwach sauren pH-Wert arbeitet.

5. Verfahren nach irgendeinem der Ansprüche 1 bis 4, dadurch gekennzeichnet, daß man einen Reaktanden der Formel (A) verwendet, gewählt aus der durch die Chloride und Bromide von Methansulfonyl, Ethansulfonyl, Phenylsulfonyl, p-Toluolsulfonyl und Trifluormethyl-sulfonyl gebildeten Gruppe.

6. Verfahren nach irgendeinem der Ansprüche 1 bis 5, dadurch gekennzeichnet, daß das verwendete Alkaliacetat das Acetat von Natrium oder Kalium ist.

7. Verfahren nach irgendeinem der Ansprüche 1 bis 6, dadurch gekennzeichnet, daß die abschließende Solvolyse mittels Natriumhydroxid oder Kaliumhydroxid in einen niederen Alkanol durchgeführt wird.

8. Verfahren nach irgendeinem der Ansprüche 1 bis 5, dadurch gekennzeichnet, daß das basische und nucleophile hydroxylierende Mittel aus der durch Natriumhydroxid, Kaliumhydroxid, Calciumhydroxid und Bariumhydroxid gebildeten Gruppe gewählt wird.

9. Verfahren nach irgendeinem der Ansprüche 1 bis 8, dadurch gekennzeichnet, daß man als Ausgangsprodukt eine Verbindung der Formel (II) verwendet, in der R₁, R₂ und R₃ einen Rest Methyl darstellen.

10. Als industrielle neue Verbindungen die Mischungen der Verbindungen der Formeln (II₁) und (III) und der Verbindungen der Formeln (II₁) und (IV) wie in Anspruch 1 definiert.

## Claims

1. Preparation process for the compounds of formula (I): 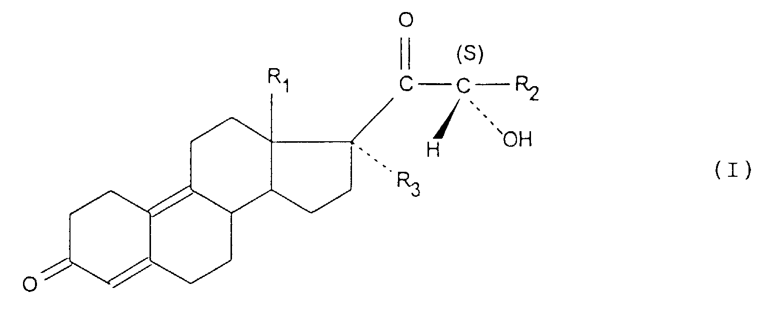 in which:
. R₁ represents an alkyl radical containing 1 to 3 carbon atoms,
. R₂ an alkyl radical containing 1 to 12 carbon atoms,
. R₃ an alkyl radical containing 1 to 4 carbon atoms
characterized in that a compound of formula (II): 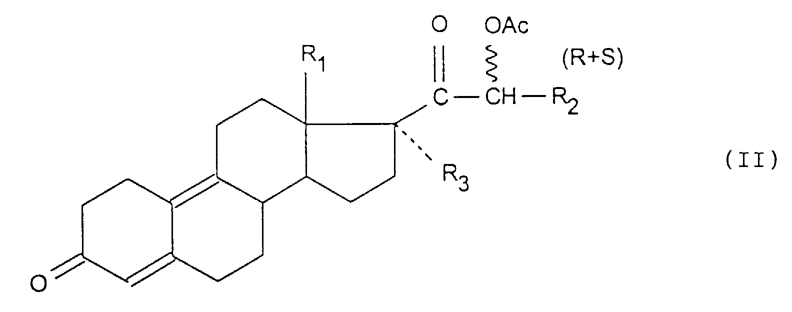 in which R₁, R₂ and R₃ are defined as previously, in the form of a mixture of isomers in position 21, is treated with an esterase or a lipase, in order to obtain a mixture of the compound of formula (III): 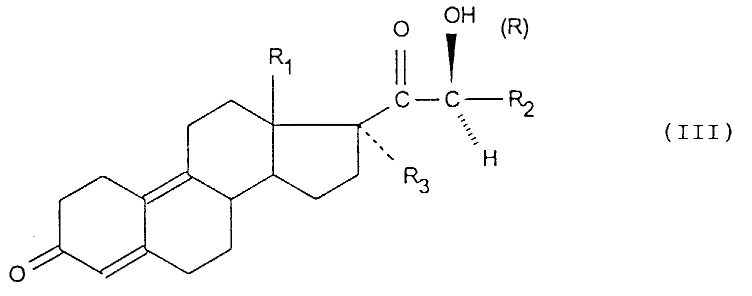 and the compound of formula (II₁): 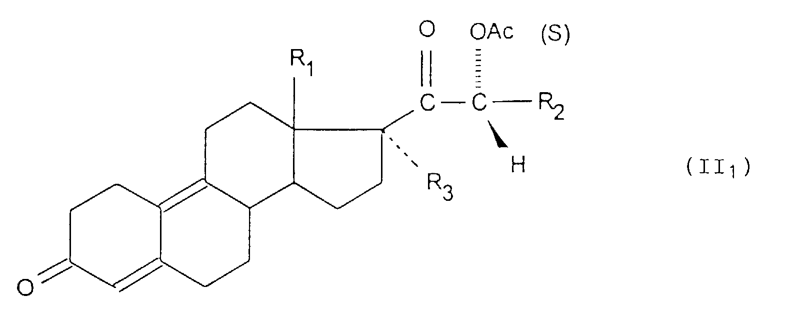 which is treated with a reagent of formula (A):
Hal-SO₂-R (A)
in which Hal represents a halogen atom and R represents an alkyl radical containing 1 to 6 carbon atoms, a phenyl radical optionally substituted by one or more alkyl radicals containing 1 to 3 carbon atoms, or a -CX₃ radical, in which X represents a fluorine, chlorine or bromine atom, in order to obtain a mixture of the compound of formula: 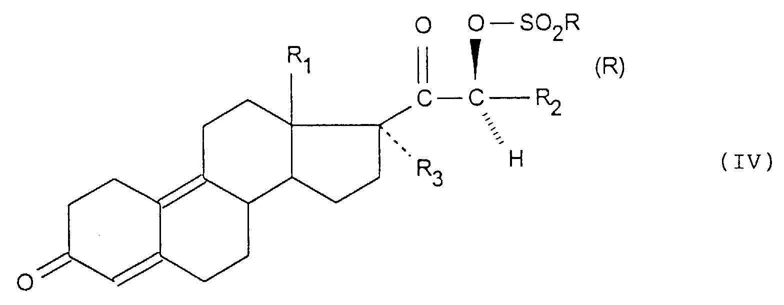 and the compound of formula (II₁) above, which is either subjected to the action of an alkaline acetate, in order to obtain the compound of formula (II₁) defined above, which is subjected to a solvolysis in a basic medium, or is subjected to the action of a basic or nucleophilic hydroxylating agent, in order to obtain the expected compound of formula (I).

2. Process according to claim 1, characterized in that the enzyme is chosen from the group constituted by Porcine Pancreatic lipase, PS "Amano", A₁ "Enzymatix", liposyme 10000 L, N "Amano", F₈ "Enzymatix", F₃ "Enzymatix", B₁ "Enzymatix" and Pseudomonas fluorescens lipase.

3. Process according to any one of claims 1 and 2, characterized in that the diastereoselective hydrolysis agent is PS "Amano" lipase.

4. Process according to any one of claims 1 to 3, characterized in that the operation takes place at a neutral or weakly acidic pH.

5. Process according to any one of claims 1 to 4, characterized in that a reagent of formula (A) is used chosen from the group constituted by methane sulphonyl, ethane sulphonyl, phenyl sulphonyl, p-toluene sulphonyl and trifluoromethyl sulphonyl chlorides and bromides.

6. Process according to any one of claims 1 to 5, characterized in that the alkaline acetate used is sodium or potassium acetate.

7. Process according to any one of claims 1 to 6, characterized in that the final solvolysis is carried out with soda or potash in a lower alkanol.

8. Process according to any one of claims 1 to 5, characterized in that the basic and nucleophilic hydroxylating agent is chosen from the group constituted by sodium hydroxide, potassium hydroxide, calcium hydroxide and barium hydroxide.

9. Process according to any one of claims 1 to 8, characterized in that a compound of formula (II) is used at the start in which R₁, R₂ and R₃ represent a methyl radical.

10. As new industrial compounds, the mixtures of the compounds of formulae (II₁) and (III) and the compounds of formulae (II₁) and (IV), as defined in claim 1.
